# FASCICULE DE BREVET EUROPEEN

(11) **EP 0 782 840 B1**
(45) Date de publication et mention de la délivrance du brevet: **01.03.2000**
(21) Numéro de dépôt: 96810855.5
(22) Date de dépôt: 06.12.1996
(51) Int. Cl.: A61B 17/16

(54) **Fraise à canon**
Fräse mit einem rohrförmigen Teil
Reamer with a tubular part

(30) Priorité: 08.01.1996 CH 4696
(43) Date de publication de la demande: 09.07.1997
(73) Titulaire: PRECIFAR S.A., 2300 La Chaux-De-Fonds (CH)
(72) Inventeur: Lechot, André, 2534 Orvin (CH)
(74) Mandataire: Kiliaridis, Constantin

(56) Documents cités:
- EP-A- 0 704 191
- FR-A- 2 281 095
- US-A- 2 785 673
- Catalogue de PRECIMED, "Fraises; gamme standard, produits spécifiques" référence 04/9804 v.1

## Description

L'invention présente a pour objet un ensemble fraise à mèche de centrage et porte-fraise destiné à la chirurgie dans le domaine de l'implantation de prothèses, tel que défini au préambule de la revendication 1.

Pour l'implantation d'une prothèse, en particulier une prothèse de la hanche, il est parfois demandé de prévoir, au fond du logement destiné à recevoir la prothèse, un trou destiné au centrage de la prothèse. A cet effet, il est connu d'utiliser des fraises munies d'une mèche pour le perçage d'un trou au centre de la partie fraisée.

Du document US-A-2 785 673 on connaît un ensemble fraise et porte-fraise selon le préambule de la revendication 1, dans lequel la fraise est munie d'un canon axial servant au montage de la fraise sur son manche et à son accouplement en rotation et dans lequel la mèche de centrage est constituée par un prolongement du manche. L'accouplement est assuré par des méplats sur le manche et dans le canon. Le canon a donc essentiellement la fonction d'un moyeu.

On connaît par ailleurs des fraises commercialisées par le demandeur sous la dénomination "fraises à cotyle coniques", dans lesquelles la mèche est soudée à même la fraise. Dans un tel outil mèche et fraise ne peuvent être interchangées, ce qui constitue un important désavantage. Pour chaque fraise doit donc être fabriquée une mèche. Dans le cas particulier des fraises coniques qui sont spécifiques à chaque patient, ceci implique une multiplicité de pièces à réaliser et donc des coûts élevés. De plus, en cas de détérioration de la fraise ou de la mèche, il n'est pas possible de les dissocier l'une de l'autre et il est nécessaire de fabriquer une nouvelle fraise et une nouvelle mèche.

Dans un autre type de fraise, la mèche est fixée de manière amovible à l'outil. En forme de calotte, ces fraises sont munies d'ergots radiaux sur leur bord intérieur, destinés à venir s'engager dans les crans d'une baïonnette. Elles sont munies au sommet de la calotte d'une ouverture destinée au passage et à l'entraînement de la mèche. Lorsqu'on utilise un telle fraise sans mèche, la fraise présente une ouverture en son sommet. Pour fraiser le centre de la partie à usiner il est donc nécessaire de faire basculer la fraise autour d'un axe perpendiculaire à son axe de rotation, ce qui rend le fraisage imprécis.

Afin d'assurer un fraisage efficace, sans basculement de la fraise, aussi proche que possible du sommet de la fraise, les bords de l'ouverture destinée au passage de la mèche peuvent être munies de dents aux arêtes latérales tranchantes. Ces dents sont toutefois non seulement beaucoup plus fragiles que les dents qui les entourent, mais elles sont les premières à attaquer le matériau à fraiser, et de ce fait très sollicitées. Le risque d'affaissement des dents, voire de cassure est important.

Si les dents sont éloignées du sommet, le fraisage au centre ne se fait pas de manière satisfaisante.

Un autre désavantage de ce type d'outil apparaît au moment de l'engagement de la mèche dans la fraise. En effet, rien n'est prévu pour guider la mèche vers l'ouverture qui lui est destinée. La mèche peut donc par erreur être engagée dans une des autres ouvertures de la fraise ce qui peut détériorer la mèche et la fraise.

Le but de la présente invention présente est de réaliser une fraise robuste dans sa zone sommitale, destinée à être utilisée avec une mèche, permettant une coupe parfaite de la partie à fraiser en particulier en son centre, et permettant une fixation simple et sûre de la mèche.

Ce but est atteint par l'ensemble fraise à mèche de centrage et porte-fraise selon l'invention telle que définie par la partie caractérisante de la revendication 1.

Un avantage particulier du canon de guidage est qu'il augmente la rigidité de la fraise.

Le montage de la mèche sur la fraise se fait de manière simple. En effet, il suffit d'introduire la mèche dans le canon de guidage jusqu'à ce que les ergots de la mèche viennent s'engager dans les encoches du canon prévues à cet effet. La fraise est ensuite montée sur le porte-fraise dont l'avancée centrale bloque axialement la mèche dans son canon de guidage et maintient les ergots de la mèche dans les encoches du canon de guidage.

Dans le cas où le fraisage ne nécessite pas la réalisation d'un trou de centrage en son milieu, une mèche plus courte, affleurant à la surface de la fraise, est avantageusement utilisée pour assurer le fraisage au sommet.

Les dents prévues à l'extrémité du canon de guidage permettent de réaliser, en particulier à proximité du centre, un fraisage parfait sans qu'il soit nécessaire de basculer la fraise (dans le cas où une mèche courte est utilisée, il est encore possible de basculer la fraise). Ceci permet au chirurgien d'obtenir une plus grande précision lors de la formation d'une cavité.

Ces dents sont solides car elles sont formées dans l'épaisseur du canon.

Le dessin annexé représente, à titre d'exemple, une forme d'exécution de l'objet de l'invention.

La figure 1 représente une vue de côté de la fraise et du porte-fraise.

La figure 2 représente une vue de dessous de la fraise de la figure 1 sans mèche.

La figure 3 représente une vue en coupe transversale de la fraise de la figure 1 sans mèche.

La figure 4 représente une vue de côté de la mèche selon l'invention.

La figure 5 représente une vue de dessus de la partie supérieure du porte-fraise de la figure 1.

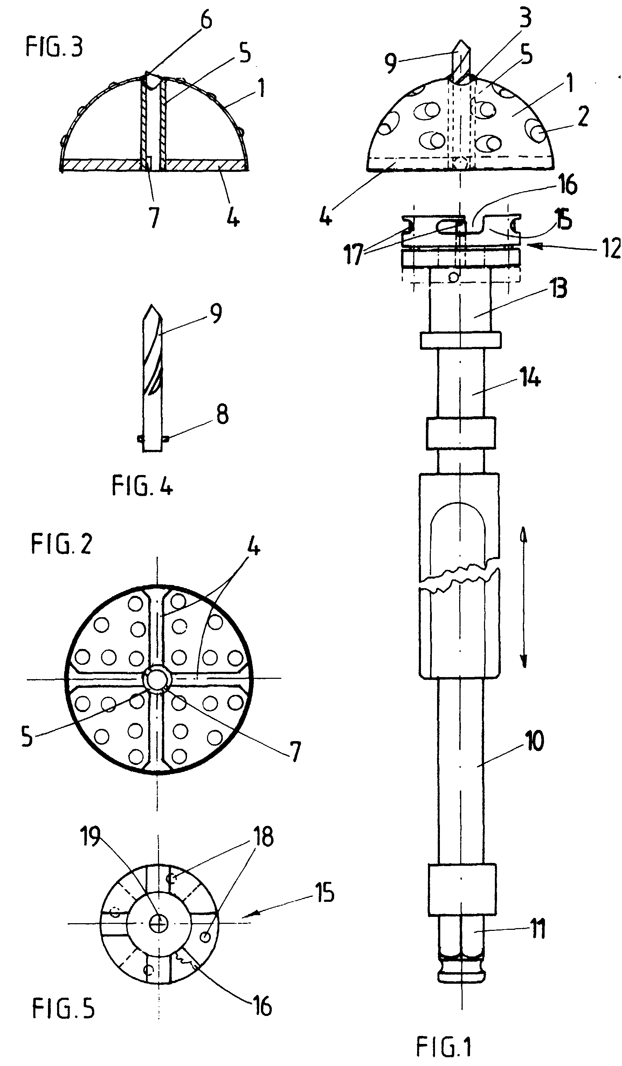

La fraise représentée aux figures 1, 2 et 3 est constituée d'une calotte hémisphérique 1 munie d'arêtes tranchantes 2 formées par perçage et repoussage du métal de la calotte, d'une ouverture 3 en son sommet et de quatre tiges radiales 4 distribuées régulièrement, dont les extrémités extérieures sont soudées au bord intérieur de la calotte et les extrémités proches du centre de la calotte 1 sont soudées sur un canon de guidage axial 5. Ce canon 5 est destiné à recevoir une mèche 9 destinée à percer un alésage central dans la cavité fraisée. Le canon de guidage 5 est constitué d'un tube cylindrique soudé par sa partie inférieure aux tiges radiales 4 et par sa partie supérieure au sommet de la fraise au bord de l'ouverture 3 de la calotte 1, le canon de guidage 5 affleurant à la surface extérieure de la calotte 1 par l'ouverture 3. Il est muni à son extrémité affleurant à la surface de la calotte 1 de deux dents 6 destinées à assurer le fraisage à proximité du sommet de la calotte et à son extrémité inférieure de deux encoches radiales 7 prévues pour recevoir deux ergots 8 de la mèche 9 introduite dans le canon de guidage 5.

Une telle mèche 9 est représentée seule en figure 4 et en position montée en figure 1. Elle est munie à sa base de deux ergots radiaux 8 destinés à être introduits dans les encoches 7 du canon de guidage 5. Une telle construction permet de rendre la mèche 9 solidaire en rotation du canon de guidage et donc de la fraise.

Le porte-fraise représenté en figure 1 comprend un manche de fraise 10, un embout 11 et une tête porte-fraise 12. L'embout 11 sert à fixer le manche 10 sur une machine l'entraînant en rotation. La tête porte-fraise 12 comprend un coulisseau 13 monté autour d'un axe 14 dont une extrémité est fixée au manche porte-fraise et dont l'autre extrémité est pourvue d'un flasque 15 de diamètre supérieur à celui de l'axe 14. Le coulisseau 13 est poussé sur l'axe 14 par un ressort qui le plaque contre le flasque supérieur 15 de l'axe 14 servant de baïonnette. Ce flasque a la forme d'une couronne dans laquelle ont été ménagés quatre crans de baïonnette 16 en forme de L destinés à recevoir les tiges radiales 4 de la fraise. Le coulisseau 13 est pourvu de quatre ergots parallèles 17 à l'axe 14, auxquels correspondent quatre trous 18 dans le flasque 15, trous que traversent les ergots 17 pour fermer les crans 16 de la baïonnette et verrouiller ainsi les tiges 4 de la fraise dans la tête du porte-fraise.

Le flasque 15 (fig. 5) est muni en son centre d'une avancée 19 destinée à bloquer la mèche 9 dans son canon de guidage 5 en appuyant les ergots 8 de la mèche 9 dans les encoches 7 du canon de guidage.

L'invention n'est pas limitée à cette forme d'exécution, mais dispose de l'étendue de protection déterminé par les revendications.

## Revendications

1. Ensemble fraise à mèche de centrage (9) et porte-fraise destiné à la chirurgie dans le domaine de l'implantation de prothèses, comprenant, d'une part, un manche (10) auquel est fixée une tête porte-fraise (12) et, d'autre part, une fraise (1) ayant une forme hémisphérique ou conique et munie d'un canon axial de guidage (5) débouchant, en affleurant, au sommet de la fraise par une ouverture (3) et destiné à recevoir la mèche de centrage (9), caractérisé en ce que la tête porte-fraise (12) est munie d'un dispositif de fixation du type baïonnette muni de moyens de verrouillage, en ce que la fraise (1) est munie de tiges radiales (4) distribuées régulièrement, solidaires du bord intérieur de la fraise (1), destinées à venir s'engager dans les crans (16) de la baïonnette, et reliées à l'extrémité inférieure du canon axial de guidage (5), en ce que l'extrémité inférieure du canon de guidage (5) est munie d'au moins une encoche (7) destinée à recevoir au moins un ergot (8) de la mèche (9) de manière à rendre le canon (5) et la mèche (9) solidaires en rotation l'un de l'autre, en ce que le porte-fraise (12) est muni en son centre d'une avancée (19) destinée à bloquer axialement la mèche (9) dans le canon de guidage (5) en appuyant l'ergot (8) de la mèche (9) dans l'encoche (7) du canon de guidage (5) lorsque la fraise (1) est montée sur le porte-fraise (12), et en ce que la partie du canon de guidage (5) solidaire du sommet de la fraise (1) est munie d'au moins deux dents de fraisage (6) affleurant au sommet de la fraise (1) par l'ouverture (3) de celui-ci, le sommet de la fraise (1) étant dégagé au niveau de ces deux dents (6) de manière à laisser libre action à celles-ci.

2. Ensemble selon la revendication 1, caractérisé en ce que la mèche a une longueur telle qu'elle affleure à la surface de la fraise en position montée de manière à assurer un fraisage au sommet de la fraise.

## Patentansprüche

1. Anordnung, bestehend aus Fräser mit zur Zentrierung dienendem Senker (9) und aus Fräserhalter, welche für chirurgische Zwecke auf dem Gebiet der Prothesenimplantation bestimmt ist, mit einerseits einem Handgriff (10), an dem ein Fräserhalterkopf (12) befestigt ist, und andererseits einem halbkugelförmigen oder konischen Fräser (1), der mit einem axialen Führungsrohr (5) versehen ist, welches mit einer Öffnung (3) am Scheitel des Fräsers glatt abschliessend mündet und dazu bestimmt ist, den Zentrierungssenker (9) aufzunehmen, dadurch gekennzeichnet, dass der Fräserhalterkopf (12) mit einer Verriegelungsmittel aufweisenden Befestigungsvorrichtung vom Bajonetttyp versehen ist, dass der Fräser (1) mit regelmässig verteilten radialen Stiften (4) versehen ist, welche fest am Innenrand des Fräsers angebracht und dazu bestimmt sind, in die Ausschnitte des Bajonetts einzugreifen, und welche mit dem unteren Ende des axialen Führungsrohrs (5) verbunden sind, dass das untere Ende des axialen Führungsrohrs (5) mit wenigstens einer Aussparung (7) versehen ist, die dazu bestimmt ist, wenigstens einen Vorsprung (8) des Senkers (9) aufzunehmen, derart, dass das Rohr (5) und der Senker (9) drehfest miteinander verbunden sind, dass der Fräserhalter (12) in seiner Mitte mit einem vorspringenden Teil (19) versehen ist, das dazu bestimmt ist, den Senker im Führungsrohr (5) axial zu blockieren, wobei sich der Vorsprung (8) des Senkers (9) in der Aussparung (7) des Führungsrohrs (5) abstützt, wenn der Fräser (1) auf dem Fräserhalter (12) montiert ist, und dass der fest am Scheitel des Fräsers (1) angebrachte Teil des Führungsrohrs (5) mit wenigstens zwei Fräserzähnen (6) versehen ist, die am Scheitel des Fräsers (1) glatt mit dessen Öffnung (3) abschliessen, wobei der Fräser (1) in Höhe dieser beiden Zähne (6) freiliegt, so dass diese frei fräsen können.

2. Anordnung nach Anspruch 1, dadurch gekennzeichnet, dass der Senker eine Länge derart hat, dass er in montierter Stellung des Fräsers glatt mit der Fräseroberfläche abschliesst, so dass ein Fräsen am Scheitel des Fräsers gewährleistet ist.

## Claims

1. A system, consisting of a cutter with a centering drill (9), and of a cutter holder, which is intended for surgical purposes in the area of prosthesis implants, comprising, on one end, a handgrip (10), on which a cutter holding head (12) is fastened, and, on the other end, a half spherical or conical cutter (1), which includes an axial guiding tube (5) opening to the apex of the cutter (1) via an opening (3) and intended to receive the centering drill (9), the system characterised in that the cutter holding head (12) is provided with a holding mechanism of the bayonet-type having a locking means, wherein the cutter (1) has evenly distributed radial rods (4) intended to engage in the cutouts (16) of the bayonets, which are firmly attached to the inner edge of the cutter and connected to the axial guide tube (5), wherein the lower end of the axial guide tube (5) is provided with at least a notch (7) to accommodate at least one projection (8) of the centering drill (9), in such a fashion that the tube (5) and the centering drill (9) are rotatably bound one with the other, wherein the cutter holding head (12) is provided in its middle with a outwardly biased part (19) which is intended to axially lock the centering drill (9) in the guide tube (5) by biasing the projection (8) of the centering drill (9) into the notch (7) of the guide tube (5) when the cutter (1) is mounted on the cutter holding head (12), and wherein the part of the guide tube (5) firmly connected with the apex of the cutter (1) is provided with at least two cutting teeth (6), opening to the apex of the cutter (1) at the said opening (3), the apex of the cutter (1) being free at the level of the two teeth (6) in a manner as to permit their free action.

2. The system according to claim 1, characterised in that the centering drill has a length such that, it opens to the surface of the cutter, in the mounted position, so that a cut at the apex of the cutter is guaranteed.
